**Europäisches Patentamt**

**European Patent Office**

**Office européen des brevets**

(19)

(11) Veröffentlichungsnummer : **0 044 491**
**B1**

(12) **EUROPÄISCHE PATENTSCHRIFT**

(45) Veröffentlichungstag der Patentschrift :
22.01.86

(21) Anmeldenummer : **81105406.3**

(22) Anmeldetag : **10.07.81**

(51) Int. Cl.⁴ : **G 01 N 33/18**, G 01 N 27/06

(54) **Verfahren zur Bestimmung des pH-Wertes von ionenarmem Wasser.**

(30) Priorität : **18.07.80 DE 3027306**

(43) Veröffentlichungstag der Anmeldung :
**27.01.82 Patentblatt 82/04**

(45) Bekanntmachung des Hinweises auf die Patenterteilung : **22.01.86 Patentblatt 86/04**

(84) Benannte Vertragsstaaten :
**AT CH DE FR GB LI SE**

(56) Entgegenhaltungen :
**EP-A- 0 013 528**
**FR-A- 2 294 444**
**FR-A- 2 428 255**
**JP-A-55 090 852**
**US-A- 3 681 025**
**US-A- 3 923 460**

(73) Patentinhaber : **Siemens Aktiengesellschaft**
**Berlin und München Wittelsbacherplatz 2**
**D-8000 München 2 (DE)**

(72) Erfinder : **Küsebauch, Walter, Dr.**
**Nötherstrasse 69**
**D-8520 Erlangen (DE)**
Erfinder : **Renner, Theodor, Dipl.-Chem.**
**Königshoferweg 17**
**D-8500 Nürnberg (DE)**

## Beschreibung

Die Erfindung betrifft ein Verfahren zur Bestimmung des pH-Wertes von ionenarmem Wasser in einem System mit einem Hauptkreislauf und mindestens einem Parallelkreislauf.

Zur Kühlung von elektrischen Einrichtungen großer Leistung, beispielsweise Generatoren und Wanderfeldröhren sowie Hochleistungskabel, sind bekanntlich geschlossene Kühlsysteme vorgesehen, die ionenarmes Kühlwasser, sogenanntes Deionat, enthalten. Das Kühlwasser durchfließt dabei die stromführenden Leiter der Einrichtung, die im allgemeinen aus Kupfer oder Kupferlegierungen bestehen. Auch zur Kühlung von besonders empfindlichen Kühlstellen, beispielsweise von Kühlköpfen und Dichtungen der Kesselumwälz- und Kesselspeisepumpen in Kraftwerken, werden bekanntlich Nebenkreisläufe geschaffen, die ebenfalls als geschlossene Kühlkreisläufe ausgeführt sind und mit einem Mischbettfilter versehen sein können. Ferner enthält der Wasser-Dampf-Kreislauf von thermischen Kraftwerken bekanntlich ionenarmes Wasser.

Um im Kühlsystem den Elektrolytgehalt möglichst niedrig zu halten, kann ein geringer Teilstrom des Kühlwassers, der beispielsweise etwa 0,5 bis 5 % betragen kann, über einen Parallelkreislauf geleitet werden, der das Mischbettfilter enthält. Das Kühlwasser erhält in solchen Kühlsystemen eine sehr geringe elektrische Leitfähigkeit, die weniger als 1 $\mu S \cdot cm^{-1}$ betragen kann. Durch das Kühlwasser kann ein Teil des Kupfers oder der Kupferlegierung durch Korrosion abgetragen und an anderen Stellen oder in nachgeschalteten Anlagenteilen wieder abgelagert werden. Die Korrosionsabtragungsrate ist abhängig vom pH-Wert des Kühlwassers und nimmt bei einem vorbestimmten pH-Wert ein Minimum an. Der pH-Wert des Kühlwassers ist somit eine wesentliche Größe für die Korrosion von Metallen.

Die elektrische Leitfähigkeit des Wassers kann mit Hilfe von Leitfähigkeitsmeßzellen gemessen werden. Die Eigendissoziation des Wassers führt zu einer elektrischen Leitfähigkeit von nur 0,064 $\mu S \cdot cm^{-1}$ bei einer Temperatur von 298 K. Diese geringe Leitfähigkeit des entsalzten Wassers ist die Ursache für Schwierigkeiten bei der pH-Messung mit Glaselektroden. So ändert die Aufnahme von Kohlendioxid den pH-Wert, wenn während der Probeentnahme Luft in das Kühlwasser gelangt. Außerdem wird das Membranglas der in pH-Meßeinrichtungen im allgemeinen verwendeten Glaselektroden an seiner Oberfläche unter Abgabe von Alkalimetallionen hydrolysiert. Ferner kann sich die Diffusionsspannung an der Bezugselektrode störend auf die pH-Messung auswirken. Die Diffusionsspannung entsteht durch die Unterschiede in den Wanderungsgeschwindigkeiten der Ionen, die an der elektrolytischen Leitung zwischen Bezugselektrode und Meßlösung beteiligt sind. Diese Diffusionsspannungen sind im entsalzten Wasser nicht immer reproduzierbar und außerdem strömungsabhängig. Darüber hinaus ist die Dissoziationskonstante des Wassers und damit auch der pH-Wert am Neutralpunkt temperaturabhängig. Die Ermittlung des pH-Wertes mit einer sogenannten stationären pH-Meßkette erfordert somit einen verhältnismäßig großen Aufwand (VGB Kraftwerkstechnik 59, Nov. 1979, Seiten 885-889).

Aus der DE-A-2 923 050 ist ein analytisches Verfahren zum Bestimmen der Konzentration von sauren oder basischen Verbindungen in einer wäßrigen Probelösung in Gegenwart von Salzen bekannt. Bei diesem Verfahren, bei dem die Säuren in einem basischen Ionenaustauscher in der Hydroxidionenform und/oder freien Aminform und die Basen in einem sauren Ionenaustauscher in der Wasserstoffionenform in Wasser umgewandelt werden und eine Leitfähigkeitsmessung erfolgt, muß ein wäßriges Trägereluiermittel verwendet werden, in welches die Probelösung eingeführt wird. Durch ein derartiges Eluiermittel kann aber eine pH-Wert-Bestimmung verfälscht werden. Darüber hinaus arbeitet das bekannte Verfahren diskontinuierlich und es erfordert Standardlösungen, was als wenig vorteilhaft anzusehen ist.

Aus der US-A-3 681 025 sind ein Verfahren und eine Vorrichtung zur Bestimmung des pH-Wertes in einer kontinuierlich fließenden Probe bekannt. Dabei wird die Probe in zwei Teile getrennt und der eine Teil durch einen Anionenaustauscher und der andere durch einen Kationenaustauscher geleitet. Hinter den beiden Ionenaustauschern wird dann jeweils die spezifische Leitfähigkeit gemessen und aus den ermittelten Leitfähigkeitswerten der pH-Wert errechnet.

Aus der JP-A-55 090 852 (entspricht der am 23-7-80 veröffentlichten) EP-A-0 013 528 sind ein Verfahren und eine Vorrichtung zur Bestimmung der Konzentration von freien Basen in Industriewässern bekannt. Dabei wird zunächst die kationische Leitfähigkeit des Wassers nach Passieren eines Ionenaustauschers gemessen und daraus die von den im Wasser gelösten Salzen herrührende Kationenkonzentration abgeleitet. Dann wird die Gesamtkonzentration der Kationen im Wasser gemessen und aus dem Unterschied zwischen der Gesamtkonzentration der Kationen und der Konzentration der aus den Salzen stammenden Kationen die Konzentration an freien Basen abgeleitet.

Der Erfindung liegt die Aufgabe zugrunde, ein Verfahren anzugeben, mit dem der pH-Wert von ionenarmem Wasser in einem System der eingangs genannten Art auf einfache Weise in weitgehender Annäherung durch Messung der elektrischen Leitfähigkeit ermittelt werden kann.

Diese Aufgabe wird erfindungsgemäß gelöst mit den Verfahrensmerkmalen nach dem Kennzeichen des Anspruchs 1.

Eine vorteilhafte Ausgestaltung des Verfahrens gemäß der Erfindung ist Gegenstand des Anspruchs 2.

Die Leitfähigkeit $\chi_1$ im Hauptkreislauf wird hervorgerufen durch Kationen und Anionen, die sich in zwei Gruppen einteilen lassen. Im basischen Bereich gehören zur ersten Gruppe die Alkaliionen und Metallionen, wie beispielsweise Kupfer-, Eisen- und Nickelionen, bei denen das $OH^-$-Anion als Partner vorliegt. Zur zweiten Gruppe gehören alle Kationen, die andere Anionen, wie beispielsweise $HCO_3^-$, $CO_3^{2-}$, $SO_4^{2-}$ sowie $Cl^-$-Ionen, als Partner haben. Die Leitfähigkeit $\chi_1$ im Hauptkreislauf läßt sich somit aufteilen in die Leitfähigkeitsanteile dieser beiden Gruppen.

Strömt das Wasser im Parallelkreislauf über den Ionenaustauscher, der im pH-Bereich $> 7$ ein Kationenaustauscher ist, so werden die Kationen der ersten Gruppe gegen $H^+$-Ionen ausgetauscht und es bildet sich zusätzliches Wasser. Die Kationen der zweiten Gruppe werden ebenfalls gegen $H^+$-Ionen ausgetauscht, sie tragen aber — ebenso wie die zugehörigen Anionen — zur Leitfähigkeit bei. Bei geringer Konzentration kann vollständige Dissoziaten angenommen werden, und mit einem Dissoziationsgrad $\sim 1$ ergibt sich die Leitfähigkeit $\chi_2$ im Parallelkreislauf aus der Ladungszahl, der Beweglichkeit und der Konzentration der Ionen. Die Grenzwerte der Ionenbeweglichkeit in wäßriger Lösung sind bekannt. Da sich die Leitfähigkeiten der Kationen zu denen der Anionen verhalten wie die Beweglichkeiten, kann man die Leitfähigkeit der $OH^-$-Ionen mit weitgehender Annäherung berechnen und erhält dann den pH-Wert als negativen Logarithmus der $H^+$-Ionenkonzentration.

Der Ionenaustauscher, der für pH-Wert-Bestimmungen im basischen Wasser mit pH-Werten $> 7$ ein Kationenaustauscher ist und für pH-Wert-Bestimmungen im sauren Wasser mit pH-Werten $< 7$ ein Anionenaustauscher, hat einen hohen Reinheitsgrad. In der Ausführungsform als Kationenaustauscher, der vorzugsweise ein stark saures Harz in der H-Form enthalten kann, hält er die Kationen zurück und gibt keine Anionen und auch keine Säurereste oder andere Verunreinigungen ab. In der Ausführungsform als Anionenaustauscher, der vorzugsweise ein stark basisches Harz in der OH-Form enthalten kann, filtert er die Anionen aus und gibt keine Kationen und keine Basenreste oder andere Verunreinigungen ab.

Soll der pH-Wert in einem System mit ionenarmem Wasser ermittelt werden, das sowohl basisch als auch sauer sein kann, so können vorzugsweise zwei Parallelkreisläufe mit jeweils einer Reihenschaltung eines Ionenaustauschers mit einer Leitfähigkeitsmeßzelle vorgesehen sein. Einer der Parallelkreisläufe enthält dann einen Kationenaustauscher und der andere einen Anionenaustauscher. Die elektrische Leitfähigkeit $\chi_3$ hinter dem Anionenaustauscher dient dann in Verbindung mit der Leitfähigkeit $\chi_1$ im Hauptkreislauf zur Bestimmung des pH-Wertes im sauren Bereich. Durch Zugabe eines Alkalisierungsmittels oder einer Säure im Hauptkreislauf und der entsprechenden Änderung der Leitfähigkeit $\chi_3$ wird bestimmt, in welchem pH-Wert-Bereich sich das Wasser befindet.

An den Meßstellen des Systems, an denen die Leitfähigkeitsmessung stattfindet, kann vorzugsweise auch die Wassertemperatur gemessen werden, die bei der pH-Wert-Bestimmung berücksichtigt werden kann.

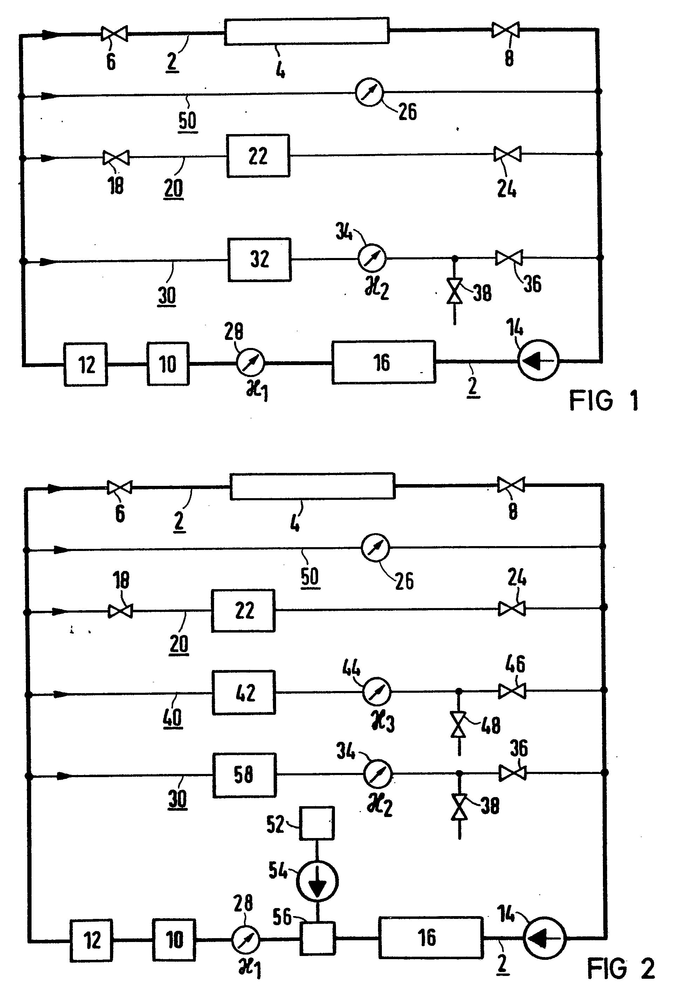

Zur weiteren Erläuterung der Erfindung wird auf die Zeichnung Bezug genommen, in der wesentliche Teile eines Kühlsystems für eine wassergekühlte Anordnung schematisch veranschaulicht sind. Figur 1 zeigt eine Ausführungsform für ein System mit einem pH-Wert, dessen Bereich bekannt ist. Eine Ausführungsform für einen pH-Wert größer oder kleiner als 7 ist in Figur 2 dargestellt. Die Anordnung wird mit ionenarmem Kühlwasser gekühlt, dessen pH-Wert mit dem Verfahren nach der Erfindung gemessen werden soll.

Nach Figur 1 ist im Hauptkreislauf 2 das Rohrsystem 4 einer elektrischen Einrichtung, beispielsweise einer wassergekühlten Generatorwicklung, in Reihe mit zwei Ventilen 6 und 8 sowie ein Magnetfilter 10 und ein mechanisches Filter 12 und eine Umwälzpumpe 14 angeordnet. Durch das Kühlwasser wird in der Generatorwicklung aufgenommene Wärme einem Wärmetauscher 16 zugeführt, der die übertragene Wärme beispielsweise an das Wasser eines Flusses abgibt. In einem Parallelkreislauf 20 ist in Reihe mit zwei Ventilen 18 und 24 ein Mischbettfilter 22 vorgesehen, das die Metallionen, beispielsweise Kupfer-, Nickel- und Eisenionen, sowie $HCO_3^-$- und $CO_3^{2-}$-Ionen aus dem Kühlwasser herausfiltert und die elektrische Leitfähigkeit auf sehr geringe Werte, beispielsweise $0{,}1 \ \mu S \cdot cm^{-1}$, vermindert. In einem weiteren Parallelkreislauf 50 kann zweckmäßig noch ein Sauerstoffmeßgerät 26 vorgesehen sein.

Die Ermittlung des pH-Wertes erfolgt mit Hilfe einer Leitfähigkeitsmeßzelle 28, die zur Messung der elektrischen Leitfähigkeit $\chi_1$ im Hauptkreislauf 2 angeordnet ist, und einer weiteren Leitfähigkeitsmeßzelle 34, die im Parallelkreislauf 30 hinter einem Ionenaustauscher 32 angeordnet ist, der zur Ermittlung von pH-Werten im Bereich größer als 7 ein Kationenaustauscher ist. Der Parallelkreislauf 30 kann durch ein Drosselventil 36 abgesperrt werden und der Teil des Wassers, der den Kationenaustauscher 32 durchflossen hat und außer $H^+$-Ionen im wesentlichen frei ist von Kationen, kann über ein Ventil 38 dem System entnommen werden. Ein Teilstrom des Kühlwassers wird über den mit Wasserstoff beladenen Kationenaustauscher 32 im Parallelkreislauf 30 geleitet, und mit der Leitfähigkeitsmeßzelle 34 wird die Leitfähigkeit $\chi_2$ in diesem Parallelkreislauf gemessen.

Die Leitfähigkeit im Hauptkreislauf wird hervorgerufen durch Kationen und Anionen, die sich in zwei Gruppen einteilen lassen. Zur ersten Gruppe gehören die $Na^+$-Ionen und Metallionen, wie beispielsweise Kupfer-, Eisen- und Nickelionen, bei denen das $OH^-$-Anion als Partner vorliegt. Zur zweiten Gruppe gehören alle Kationen, die ein anderes Anion, wie beispielsweise $HCO_3^-$, $CO_3^{2-}$ und $SO_4^{2-}$ sowie $Cl^-$, als Partner haben.

Die Leitfähigkeit $\chi_1$ des Wassers im Hauptkreislauf läßt sich somit aufteilen in die Leitfähigkeitsanteile der ersten Gruppe, nämlich den Leitfähigkeitsanteil $\chi_{K1}$ der Alkaliionen und Metallionen und den Leitfähigkeitsanteil $\chi_{OH^-}$ der zugehörigen $OH^-$-Ionen, und die Leitfähigkeitsanteile der zweiten Gruppe,

nämlich den Leitfähigkeitsanteil $\chi_{K2}$ der Kationen der zweiten Gruppe und den Leitfähigkeitsanteil $\chi_A$ der zugehörigen Anionen. Weiterhin trägt die Eigendissoziation des Wassers mit einem Leitfähigkeitsanteil zur Leitfähigkeit bei.

Strömt dieses Wasser über den wasserstoffbeladenen Kationenaustauscher 32, so werden die Kationen der ersten Gruppe gegen $H^+$-Ionen ausgetauscht und es bildet sich Wasser. Die Kationen der zweiten Gruppe werden ebenfalls gegen $H^+$-Ionen ausgetauscht, tragen aber ebenso wie die zugehörigen Anionen zur Leitfähigkeit des Wassers bei. Die im Parallelkreislauf 30 hinter dem Kationenaustauscher 32 gemessene elektrische Leitfähigkeit $\chi_2$ ergibt sich somit aus dem Leitfähigkeitsanteil $\chi_H$ der $H^+$-Ionen und dem Leitfähigkeitsanteil $\chi_A$ der Anionen.

Die Leitfähigkeiten der Kationen und der Anionen verhalten sich zueinander wie ihre Beweglichkeiten, weil die Konzentration der Kationen gleich der Konzentration der Anionen ist. Weiterhin kann bei kleinen Konzentrationen mit vollständiger Dissoziation gerechnet werden. Man kann somit aus den Ionenbeweglichkeiten $I_H$ der $H^+$-Ionen, $I_{OH}$ der $OH^-$-Ionen, $I_K$ der Kationen sowie $I_A$ der Anionen und der Leitfähigkeit $\chi_2$ die einzelnen Leitfähigkeitsanteile berechnen und man erhält den Leitfähigkeitsanteil $\chi_{OH}$ aus der Beziehung

$$\chi_1 = \left(\frac{I_K}{I_{OH}} + 1\right) \cdot \chi_{OH} + \frac{I_K + I_A}{I_A + I_H} \cdot \chi_2 \ \text{zu}$$

$$\chi_{OH} = \frac{\chi_1 - \dfrac{I_K + I_A}{I_A + I_H} \cdot \chi_2}{\dfrac{I_K}{I_{OH}} + 1}$$

Mit Hilfe des Ionenproduktes $K_w$ des Wassers und der Definition des pH-Wertes als negativer Logarithmus der $H^+$-Ionenkonzentration erhält man den pH-Wert zu

$$pH = -\log \frac{(I_K + I_{OH}) \cdot K_w}{1\,000} + \log \left(\chi_1 - \frac{I_K + I_A}{I_A + I_H} \cdot \chi_2\right).$$

Die Beweglichkeit der Ionen und das Ionenprodukt $K_w$ des Wassers sowie die Leitfähigkeiten sind temperaturabhängig. Diese Temperaturabhängigkeit ist bekannt. Die Temperatur wird an den Leitfähigkeitsmeßzellen gemessen und kann vorzugsweise auf wenigstens annähernd dem gleichen Wert gehalten werden. Für eine Temperatur von 298 K beispielsweise erhält man mit einer mittleren Kationenbeweglichkeit, beispielsweise der $Na^+$-Ionen sowie $Cu^{2+}$-, $Fe^{2+}$- und $Ni^{2+}$-Ionen, von 52 $\Omega^{-1} \cdot cm^2$ und einer mittleren Anionenbeweglichkeit, beispielsweise der $HCO_3^-$-, $CO_3^{2-}$-, $SO_4^{2-}$- und $Cl^-$-Ionen, von $62 \Omega^- \cdot cm^2$ sowie einer Beweglichkeit der $H^+$-Ionen $I_H$ von 349,8 $\Omega^{-1} \cdot cm^2$ und einer Beweglichkeit der $OH^-$-Ionen $I_{OH}$ von 198,6 $\Omega^{-1} \cdot cm^2$ sowie mit dem Wert des Ionenprodukts von Wasser, $K_w = 1{,}008 \cdot 10^{-14}$, den pH-Wert aus der Beziehung

$$pH = 14{,}60 + \log (\chi_1 - 0{,}2768\,\chi_2).$$

Die Abweichung des sich ergebenden pH-Wertes vom wirklichen pH-Wert beträgt beispielsweise für pH-Werte > 8,1 weniger als 1 %.

Für eine Bestimmung des pH-Wertes im sauren Bereich wird als Ionenaustauscher 32 ein Anionenaustauscher gewählt. Hinter dem Anionenaustauscher wird eine abweichende elektrische Leitfähigkeit $\chi_3$ gemessen und man erhält den pH-Wert im Bereich < 7 durch entsprechende Ableitung in weitgehender Annäherung aus der Beziehung

$$pH = \log \frac{I_H + I_A}{1\,000} - \log \left(\chi_1 - \frac{I_K + I_{OH}}{I_K + I_{OH}} \cdot \chi_3\right)$$

Für eine Temperatur beispielsweise von 298 K an den Meßstellen der elektrischen Leitfähigkeit ergibt sich der pH-Wert aus der vereinfachten Beziehung

$$pH = -0{,}3853 - \log (\chi_1 - 0{,}4549\,\chi_3).$$

Die Abweichung des sich ergebenden pH-Wertes vom wirklichen pH-Wert beträgt im sauren Bereich beispielsweise für pH-Werte < 6,1 wesentlich weniger als 1 %.

Falls nicht bekannt ist, ob das ionenarme Wasser, dessen pH-Wert ermittelt werden soll, im basischen pH-Wert-Bereich > 7 oder im sauren pH-Wert-Bereich < 7 liegt, kann eine pH-Wert-Bestimmung mit der Anordnung nach Figur 2, in welcher der Figur 1 entsprechende Teile mit den gleichen Bezugsziffern versehen sind, durchgeführt werden. Das System soll ebenfalls zur Kühlung einer Generatorwicklung vorgesehen sein, deren Kühlrohrsystem 4 zusammen mit einem Mangetfilter 10, einem mechanischen Filter 12 und einer Umwälzpumpe 14 sowie einem Wärmetauscher 16 im Hauptkreislauf 2 angeordnet ist. Die Anordnung enthält im Parallelkreislauf 20 in Reihe mit Ventilen 18 und 24 ein Mischbettfilter 22. Der zur Messung der Leitfähigkeit $\chi_2$ im alkalischen Bereich dienende Parallelkreislauf 30 enthält einen Kationenaustauscher 58 und eine Leitfähigkeitsmeßzelle 34 sowie Ventile 36 und 38.

Die Ermittlung des pH-Wertes erfolgt durch Messung der Leitfähigkeit $\chi_1$ im Hauptkreislauf 2 und durch Messung der Leitfähigkeit $\chi_2$ im Parallelkreislauf 30 sowie durch Messung der Leitfähigkeit $\chi_3$ in einem zusätzlichen Parallelkreislauf 40 mit Hilfe einer Leitfähigkeitsmeßzelle 44, die hinter einem Anionenaustauscher 42 in Reihe mit einem Ventil 46 angeordnet ist. Der Parallelkreislauf 40 enthält auch ein Ventil 48 zum Ablassen von Kühlwasser.

Mit einer Zuführung 56 kann dem Hauptkreislauf 2 aus einem Behälter 52 über eine Dosierpumpe 54 ein Alkalisierungsmittel oder gegebenenfalls eine Säure in verdünnter Form zugeführt und zunächst durch die Änderung der Leitfähigkeit $\chi_1$ des Wassers festgestellt werden, in welchem Bereich sich der pH-Wert des Wassers befindet. Wird ein Alkalisierungsmittel in den Hauptkreislauf eingeführt und die Leitfähigkeit $\chi_1$ steigt an, so liegt ein pH-Wert im basischen Bereich vor. Wird eine Säure zugeführt und die Leitfähigkeit $\chi_1$ des Wassers steigt, so liegt der pH-Wert im sauren Bereich. Sinkt die Leitfähigkeit $\chi_1$, so liegt der pH-Wert im basischen Bereich.

Hat man beispielsweise den basischen pH-Wert-Bereich $> 7$ festgestellt, so erfolgt die Ermittlung des pH-Wertes durch Messung der Leitfähigkeiten $\chi_1$ und $\chi_2$ und Ableitung über die Ionenbeweglichkeiten. Im sauren pH-Wert-Bereich $< 7$ erfolgt die Ermittlung des pH-Wertes entsprechend durch Messung der Leitfähigkeiten $\chi_1$ und $\chi_3$.

## Patentansprüche

1. Verfahren zur Bestimmung des pH-Wertes von ionenarmem Wasser in einem System mit einem Hauptkreislauf (2) und mindestens einem Parallelkreislauf, dadurch gekennzeichnet, daß die elektrische Leitfähigkeit $\chi_1$ des Wassers im Hauptkreislauf (2) und die elektrische Leitfähigkeit $\chi_2$ bzw. $\chi_3$ des Wassers im Parallelkreislauf (30) hinter einem Ionenaustauscher (32) gemessen wird, wobei im basischen Bereich der Ionenaustauscher (32) ein Kationenaustauscher ist und $\chi_2$ gemessen wird und im sauren Bereich der Ionenaustauscher (32) ein Anionenaustauscher ist und $\chi_3$ gemessen wird, und daß der pH-Wert aus den Leitfähigkeitsanteilen der einzelnen Ionengruppen sowie den Ionenbeweglichkeiten und aus dem Ionenprodukt des Wassers ermittelt wird, und zwar im basischen Bereich nach der Formel

$$pH = -\log\frac{K_w(l_K + l_{OH})}{1\,000} + \log\left(\chi_1 - \frac{l_K + l_A}{l_A + l_H}\kappa\,\chi_2\right)$$

und im sauren Bereich nach der Formel

$$pH = \log\frac{l_A + l_H}{1\,000} - \log\left(\chi_1 - \frac{l_K + l_A}{l_K + l_{OH}}\cdot\chi_3\right)$$

wobei die Ionenbeweglichkeiten in $\Omega^{-1}\cdot cm^2$ und die Leitfähigkeiten in $S\cdot cm^{-1}$ einzusetzen sind.

2. Verfahren nach Anspruch 1, dadurch gekennzeichnet, daß der basische bzw. saure pH-Wert-Bereich durch Zugabe einer Lauge oder Säure zum Wasser und aus dem Anstieg bzw. dem Abfall der elektrischen Leiftähigkeit $\chi_1$ im Hauptkreislauf (2) ermittelt wird, daß die elektrische Leitfähigkeit $\chi_3$ in einem zusätzlichen Parallelkreislauf (40) hinter einem Anionenaustauscher (42) gemessen wird und daß die Leitfähigkeit $\chi_2$ im Parallelkreislauf (30) hinter einem Kationenaustauscher (58) gemessen wird.

## Claims

1. Method for measuring the pH-value of weakly ionised water, in a system having a main cycle (2) and at least one parallel cycle, characterised in that the electrical conductivty $\chi_1$ of the water in the main cycle (2) and the electrical conductivity $\chi_2$ or $\chi_3$ of the water in the parallel cycle (30) is measured downstream of an ion exchanger (32), whereby in the basic region the ion exchanger (32) is a cation exchanger and $\chi_2$ is measured, and in the acid region the ion exchanger (32) is an anion exchanger and $\chi_3$ is measured ; and that the pH-value is determined from the conductivity components of the individual ion groups and the ion mobilities and from the ion product of the water, in the basic region in accordance with the formula

$$pH = -\log\frac{K_w(l_K + l_{OH})}{1\ 000} + \log\left(\chi_1 - \frac{l_K + l_A}{l_A + l_H}\,^K\,\chi_2\right)$$

and in the acid region in accordance with the formula

$$pH = \log\frac{l_A + l_H}{1\ 000} - \log\left(\chi_1 - \frac{l_K + l_A}{l_K + l_{OH}} \cdot \chi_3\right),$$

where the ion mobilities are given in $\Omega^{-1} \cdot cm^2$ and the conductivities in $S \cdot cm^{-1}$.

2. A method as claimed in Claim 1, characterised in that the basic or acid pH-value region is determined by adding a base or an acid to the water and from the rise or fall, as the case may be, in the electrical conductivity $\chi_1$ in the main cycle (2) ; that the electrical conductivity $\chi_3$ is measured in an additional parallel cycle (40) downstream of an anion exchanger (42) ; and that the conductivity $\chi_2$ in the parallel cycle (30) is measured downstream of a cation exchanger (58).

**Revendications**

1. Procédé de détermination de la valeur du pH d'eau faiblement ionisée dans un système comprenant un circuit principal (2) et au moins un circuit parallèle, caractérisé en ce qu'il consiste à mesurer la conductibilité électrique $\chi_1$ de l'eau dans le circuit principal (2) et la conductibilité électrique $\chi_2$ ou $\chi_3$ de l'eau dans le circuit parallèle (30), en aval d'un échangeur d'ions (32), alors que dans le domaine basique, l'échangeur d'ions (32) est un échangeur de cations et que l'on mesure $\chi_2$ et que dans le domaine acide l'échangeur d'ions (32) est un échangeur d'anions et que l'on mesure $\chi_3$, et à déterminer la valeur du pH à partir des quotes-parts des conductibilités des groupes d'ions individuels, ainsi que des mobilités des ions et du produit ionique de l'eau, et cela, dans le domaine basique, suivant la formule

$$pH = -\log\frac{K_w(l_K + l_{OH})}{1\ 000} + \log\left(\chi_1 - \frac{l_K + l_A}{l_A + l_H}\,^K\,\chi_2\right)$$

et dans le domaine acide suivant la formule

$$pH = \log\frac{l_A + l_H}{1\ 000} - \log\left(\chi_1 - \frac{l_K + l_A}{l_K + l_{OH}} \cdot \chi_3\right)$$

les mobilités ioniques étant exprimées en $\Omega^{-1} \cdot cm^2$ et les conductibilités en $S \cdot cm^{-1}$.

2. Procédé suivant la revendication 1, caractérisé en ce qu'il consiste à déterminer le domaine basique ou acide de la valeur du pH par addition d'une lessive ou d'un acide à l'eau, et à partir de l'augmentation ou de la diminution de la conductibilité électrique $\chi_1$ dans le circuit principal (2), à mesurer la conductibilité électrique $\chi_3$ dans un circuit parallèle (40) supplémentaire en aval d'un échangeur d'anions (42), et à mesurer la conductibilité $\chi_2$ dans le circuit parallèle (30) en aval d'un échangeur de cations (58).

0 044 491

FIG 1

FIG 2

1